# EUROPEAN PATENT APPLICATION

(11) **EP 4 600 347 A1**
(43) Date of publication of application: **13.08.2025**
(21) Application number: 25156715.2
(22) Date of filing: 07.02.2025
(51) Int. Cl.: C12N 5/071

(54) **A SYSTEM AND COMPOSITION OF RESPIRATORY ORGANOIDS FOR PROPAGATION OF VIRUSES AND METHODS THEREOF**

(30) Priority: 08.02.2024 US 202463551522 P
(71) Applicant: The University of Hong Kong, Hong Kong (HK); Centre for Virology, Vaccinology and Therapeutics Limited, Pak Shek Kok, N.T. (HK)
(72) Inventor: LI, Cun, Hong Kong (HK); YU, Yifei, Hong Kong (HK); ZHOU, Jie, Hong Kong (HK); CHIU, Man Chun, Hong Kong (HK)
(74) Representative: HGF

(57) **Abstract**

The disclosure is generally directed to a composition, method and system of respiratory organoids that sustain propagation of respiratory pathogens. Also provided is a method of modeling a biological process in a human lung. Also provided is a method of screening for compounds that affect respiratory pathogens in a respiratory organoid model.

## Description

### RELATED APPLICATIONS

The present application claims priority to U.S. Provisional Application Serial No. 63/551,522 filed February 8, 2024 which is incorporated by reference in its entirety.

### 1. FIELD

The disclosure is generally directed to a system, composition and method of respiratory organoids that sustain propagation of respiratory pathogens. Also provided is a method of modeling a biological process in a human lung. Also provided is a method of screening for compounds that affect respiratory pathogens in a respiratory organoid model.

### 2. BACKGROUND

Human rhinovirus (HRV) is a nonenveloped, positive-stranded RNA virus of the Piconaviridae family and the Enterovirus genus, grouped into three species (HRV-A, HRV-B, and HRV-C)¹. HRV is the most common pathogen of acute upper respiratory infection, linked with lower respiratory infections and exacerbation of asthma and chronic pulmonary diseases. HRV-A and HRV-B are composed of around 100 serotypes and can be readily propagated in immortalized cell lines, using intercellular adhesion molecule 1 (ICAM-1) and low-density lipoprotein receptor (LDLR) for cellular entry. Since HRV-C was identified in 2006², over 60 distinct HRV-C sequences have been reported so far by molecular approaches³. In contrast to HRV-A and HRV-B, HRV-C cannot infect and replicate in standard cell lines. Tremendous efforts have been engaged to tackle the problem during the past 2 decades. Bochkov et al reported the first *in vitro* HRV-C infection in primary tissues from surgically resected sinus mucosa and nasal polyps, and they successfully used these tissues to propagate a single HRV-C15 strain. However, virus growth exhibited significant variations, which may be related to the tissues from different donors, and variable status of tissues⁴. Using primary tissues for virus cultivation has presented several challenges, including limited accessibility of the tissues, the difficulty of standardization, and tissue degradation during experimentations⁵. Subsequently, the same team identified CDHR3 as the cellular receptor of HRV-C⁶, a landmark discovery in HRV-C research.

HRV-C infection and limited propagation were also reported in primary human airway epithelial cells^{7,8}. Air-liquid interface cultures of the primary human airway and nasal epithelial cells allow mucociliary differentiation into the pseudostratified ciliated epithelium. These primary epithelial cells have been used to delineate respiratory viral infections, including CO VID-19^{9,10}. Yet the limited expansion capacity inherent to primary epithelial cells substantially restricts their application for routine experimentations, including virus isolation and serial virus passage. Moreover, HRV-C is not the sole uncultivable virus. Other human viruses of respiratory tropism, such as bocavirus¹¹ and human coronavirus HKU1¹², are unable to infect immortalized cell lines, except in *ex vivo* respiratory tissues and primary airway epithelial cells; these human viruses have yet to be cultivated for comprehensive investigations. Therefore, there is an unmet need for a robust and readily accessible cultivation system to propagate these uncultivable viruses, which is the initial step for understanding these pathogenic microbes. Overall, the lack of a robust cultivation system represents a major hurdle to understanding the biology of HRV-C and other uncultivable respiratory viruses, their interaction with human respiratory cells, and the development of antiviral strategies against these common respiratory pathogens.

Organoids, also called "mini-organs," are three-dimensional (3D) cellular clusters derived from stem cells, including embryonic stem cells, induced pluripotent stem cells, and organ-specific adult stem cells in primary tissues. They can self-renew and self-organize into complex, functional structures that recapitulate the cellular diversity and physiological functions of corresponding tissues and organs.

### 3. SUMMARY

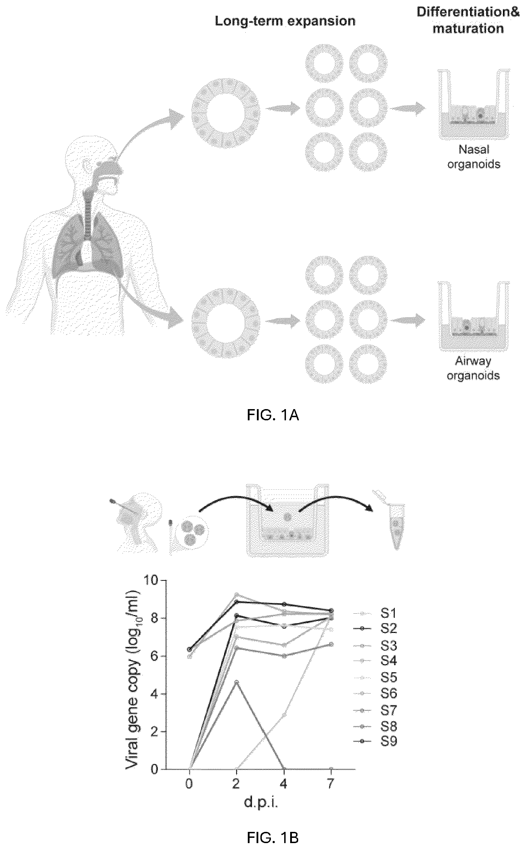

We have established the first human respiratory organoid culture system¹³⁻¹⁹ Organoids are derived from adult stem cells in primary lung tissues with high efficiency and can be stably expanded over half a year. We have developed differentiation protocols to induce maturation in long-term expandable organoids and generate mature airway organoids and alveolar organoids that faithfully simulate the native airway and alveolar epithelium, respectively²⁰. Apart from a noninvasive procedure to procure readily accessible nasal cells for organoid derivation, nasal organoids more adequately simulate the upper airway epithelium than airway organoids¹⁷. In this two-phase organoid culture system^{17,20}, expansion culture provides a stable and long-term expanding source, while differentiation protocols enable us to generate large amounts of physiologically active respiratory epithelial cells (FIG. 1A). Thus, the respiratory organoid culture system allows us to rebuild and propagate the entire human respiratory epithelium in culture plates with excellent efficiency and stability. These respiratory organoids have become robust and popular tools for studying SARS-CoV-2 and other respiratory viruses ^{16,17,21,23}.

Given the respiratory tropism of HRV-C and the ability of nasal and airway organoids to accurately simulate the native epithelium in human airways, we hypothesized that these respiratory organoids (refer to airway and nasal organoids hereafter) may be susceptible to HRV-C and sustain virus cultivation. We were prompted to develop an organoid-based culture system to reproducibly propagate HRV-C and characterize HRV-C infection and complex virus-host interaction in these respiratory organoids.

Provided is a composition for culturing a respiratory microorganism, the composition comprising: (i) respiratory organoids selected from the group consisting of airway organoids and nasal organoids; and (ii) a respiratory microorganism, wherein the respiratory microorganism sustains serial propagation in the respiratory organoids.

Provided is a method for culturing respiratory microorganisms comprising: (i) preparing respiratory organoids, wherein the respiratory organoids are selected from the group consisting of airway organoids and nasal organoids; and (ii) infecting the respiratory organoids with a microorganism, wherein the microorganism sustains propagation in the respiratory organoid.

Provided is a system for culturing respiratory microorganisms comprising: (i) a fragment of lung tissue in a culture medium comprising a JAK1/JAK2 inhibitor or a TBK1/IKKε inhibitor cultured for a period of time sufficient to form respiratory organoids; and (ii) respiratory microorganisms that sustain serial propagation in the respiratory organoids.

Provided herein is a new human respiratory organoid sustained reproducible propagation of human rhinovirus C.

Provided is an organoid-based system and composition to reproducibly propagate the previously uncultivable HRV-C, which allowed us to elucidate HRV-C infection and innate immunity in an unprecedented manner. The organoid-based HRV-C infection model can be extended for developing antiviral strategies. In one embodiment, provided is an avenue for propagating and studying other uncultivable human and animal viruses.

Provided is a human respiratory organoid culture system and composition to rebuild and propagate the entire human respiratory epithelium in culture plates with excellent efficiency and stability. The principle rationale of airway and nasal organoids for isolation and propagation of uncultivable viruses lies in their high biological relevance to the native respiratory epithelium, the primary target of many viruses, including those viruses uncultivable in standard cell lines.

Provided is a system, composition and a method by which previously uncultivable or poorly cultivable viruses are reproducibly cultivated and propagated.

Provided is a system, composition and a method of biologically active airway and nasal organoids for isolating and cultivating previously uncultivable human respiratory viruses.

Provided are airway organoids and methods that sustain serial virus propagation with the aid of CYT387-mediated immunosuppression, nasal organoids that more closely simulate the upper airway achieve this without any intervention.

Also provided are systems, compositions and methods useful as research tools for identifying agents effective in treating respiratory diseases and infections. Also provided is a composition comprising a respiratory organoid model culture comprising human respiratory organoids including alveolar, airway and nasal organoids. In certain embodiments, the organoids comprise epithelial cells, mesenchymal and immune cells. Also provided are methods of infecting the composition with a respiratory microorganism and serial propagation of the microorganisms in the culture. The methods further include screening for a compound that affects the microorganisms. The methods further comprise administering a compound to the composition and determining whether a treatment effective response is present. In one embodiment, a method of identifying a therapeutic agent effective for treating a respiratory disease and infections due to pathogens is provided.

In one embodiment, provided is an organoid chip which is a hybrid device with the form of a conventional semiconductor chip made by combining organoids and inorganic materials such as semiconductors or glass. By utilizing the unique functions of biomolecules and mimicking the functions of living organisms, it has the characteristic of diagnosing infectious diseases, analyzing genes, and serving as a new functional device for new information processing. In certain embodiments, the organoid chip comprises a biosensor that can detect and analyze various biochemical substances, such as a lab on a chip with an automatic analysis function, by compactly integrating sample pretreatment, biochemical reaction, detection, and data analysis, and can be broadly defined.

Provided is a composition for culturing a respiratory microorganism, the composition comprising: (i) respiratory organoids selected from the group consisting of airway organoids and nasal organoids; and (ii) a respiratory microorganism, wherein the respiratory microorganism sustains serial propagation in the respiratory organoids.

In one embodiment, the respiratory organoids are airway organoids and derived from lung tissue.

In one embodiment, the respiratory organoids are nasal organoids and derived from nasal epithelial cells.

In one embodiment, the respiratory microorganism sustains at least 3-6 passages with genomic stability.

In one embodiment, the composition further comprising an immunosuppressive compound.

In one embodiment, the immunosuppressive compound interferes with an anti-microorganism response in the respiratory organoids.

In one embodiment, the immunosuppressive compound is a JAK1/JAK2 inhibitor or a TBK1/IKKε inhibitor.

In one embodiment, the immunosuppressive compound is Momelotinib (CYT387), Amlexanox, BX795, MRT67307, Ruxolitinib or a combination thereof.

In one embodiment, the respiratory microorganism is a respiratory virus or a respiratory bacteria.

In one embodiment, the respiratory virus is HRV-C, HRV-C3, HRV-C8, HRV-C11, HRV-C15, HRV-C45, influenza virus, adenovirus, human bocavirus, human coronavirus including SARS-CoV1 and SARS-CoV2, human metapneumovirus, human parainfluenza virus, human respiratory syncytial virus, or human rhinovirus.

In one embodiment, the respiratory bacteria is *Mycobacterium tuberculosis, Streptococcus pneumoniae, Mycoplasma pneumoniae, Hemophilus influenzae, Chlamydophila pneumoniae, Chlamydia psittaci, Coxiella burnetiid, Legionella pneumophila, Staphylococcus aureus,* or *Klebsiella pneumoniae.*

In one embodiment, the respiratory organoids are human respiratory organoids comprising human respiratory epithelial cells.

In one embodiment, the respiratory organoids comprise human lung epithelial cells.

In one embodiment, the respiratory organoids comprise human nasal epithelial cells.

In one embodiment, the respiratory organoids are derived from human epithelial stem cells.

In one embodiment, the respiratory organoids are viable in culture for a period of at least 30 days.

Provided is a method for culturing respiratory microorganisms comprising: (i) preparing respiratory organoids, wherein the respiratory organoids are selected from the group consisting of airway organoids and nasal organoids; and (ii) infecting the respiratory organoids with a microorganism, wherein the microorganism sustains propagation in the respiratory organoid.

In one embodiment, the method further comprising a step of isolating and cultivating the respiratory microorganisms.

In one embodiment, the respiratory organoids are airway organoids derived from lung tissue.

In one embodiment, the respiratory organoids are nasal organoids derived from nasal epithelial cells.

In one embodiment, the respiratory organoids are prepared by (a) providing a fragment of an airway tissue or nasal tissue; and (b) culturing the fragment of airway or nasal tissue in a culture medium for a period of time sufficient to form the respiratory organoids.

In one embodiment, the culture medium further comprises an immunosuppressive compound.

In one embodiment, the immunosuppressive compound interferes with an anti-microorganism response in the respiratory organoids.

In one embodiment, the immunosuppressive compound is a JAK1/JAK2 inhibitor or a TBK1/IKKε inhibitor.

In one embodiment, the immunosuppressive compound is Momelotinib (CYT387), Amlexanox, BX795, MRT67307, Ruxolitinib or a combination thereof.

In one embodiment, the respiratory microorganism sustains at least 3-6 passages with genomic stability.

In one embodiment, the respiratory microorganism is a respiratory virus or a respiratory bacteria.

In one embodiment, the respiratory virus is HRV-C, HRV-C3, HRV-C8, HRV-C11, HRV-C15, HRV-C45, influenza virus; adenovirus; human bocavirus; human coronavirus including SARS-CoV1 and SARS-CoV2; human metapneumovirus; human parainfluenza virus; human respiratory syncytial virus; and/or human rhinovirusor a combination thereof.

In one embodiment, the respiratory bacteria is *Mycobacterium tuberculosis, Streptococcus pneumoniae, Mycoplasma pneumoniae, Haemophilus influenzae, Chlamydophila pneumoniae; Chlamydia psittaci; Coxiella burnetiid; Legionella pneumophila, Staphylococcus aureus;* and/or *Klebsiella pneumoniae.*

In one embodiment, the respiratory organoids are human respiratory organoids comprising human respiratory epithelial cells.

In one embodiment, the respiratory organoids are viable in culture for a period of at least 30 days.

Provided is a system for culturing respiratory microorganisms comprising: (i) a fragment of lung tissue in a culture medium comprising a JAK1/JAK2 inhibitor or a TBK1/IKKε inhibitor cultured for a period of time sufficient to form respiratory organoids; and (ii) respiratory microorganisms that sustain serial propagation in the respiratory organoids.

### 4. BRIEF DESCRIPTION OF THE FIGURES

The patent or application file contains at least one drawing executed in color. Copies of this patent or patent application publication with color drawing(s) will be provided by the Office upon request and payment of the necessary fee.
FIGs. 1A-C. Human airway organoids were susceptible to clinical specimens of HRV-C. (A) A schematic illustration of the human nasal organoid and airway organoid culture system. (B) Airway organoids (in 9 transwell inserts) were inoculated with 9 HRV-C+ nasopharyngeal aspirates. At the indicated day post-infection (d.p.i.), culture media were harvested from the infected airway organoids and applied to viral load detection by RT-qPCR. A schematic graph of the experimental procedure was created with Biorender.com. (C) Airway organoids were inoculated with HRV-A1 and HRV-C3 at 100 viral gene copy/cell (n=3). Culture media were harvested from apical and basolateral chambers of infected airway organoids at the indicated d.p.i. and applied to the viral load detection. Data represent mean and SD of the indicated number (n) of biological replicates from a representative experiment independently performed three times. Statistical significance (in panel C) was determined using a two-tailed Student's t-test. **P < 0.01, ***P < 0.001.

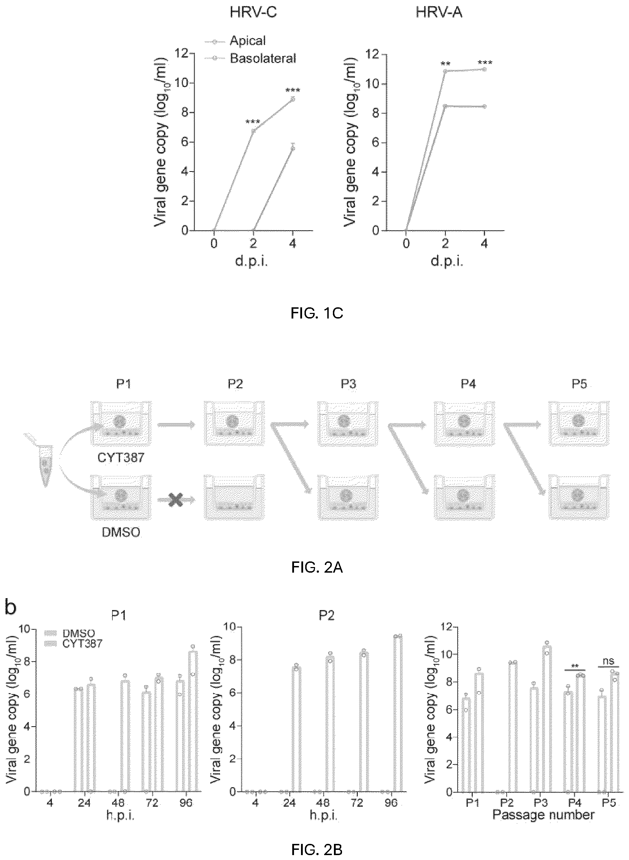

FIGs 2A-C. CYT387 enabled serial propagation of HRV-C in human airway organoids. (A) A schematic graph outlines the experimental procedure for panels B. (B) In the first (P1) passage, after pre-treatment with CYT387 or DMSO overnight, airway organoids were inoculated with HRV-C3 (n=2). Culture media were harvested from infected airway organoids at indicated h.p.i. to detect viral replication. In the second (P2) passage, airway organoids pretreated with CYT387 or DMSO were inoculated with 50µl P1 medium collected from CYT387- or DMSO-treated organoids, respectively (n=2). Culture media were harvested at indicated h.p.i. to detect viral replication. From P3, airway organoids pretreated with CYT387 or DMSO were inoculated with medium collected from CYT387-treated organoids at 100 viral gene copy/cell (P3, n=2; P4 and P5, n=3). The culture media were harvested at 96 h.p.i. to detect viral replication. The viral loads in P1 and P2 media at 96 h.p.i. are incorporated. Data represent mean and SD of the indicated number (n) of biological replicates from a representative experiment. Statistical significance (P4 and P5) was determined using a two-tailed Student's t-test. **P < 0.01. *ns* not significant. (C) At 24 h.p.i. of HRV-C3, airway organoids were fixed and immune-labeled with an α-VP3 (green) and α-ACCTUB (red). Nuclei and actin filaments were counterstained with DAPI (blue) and Phalloidin-647 (white), respectively. The experiment was independently performed three times with similar results. Scale bar, 10 µm.

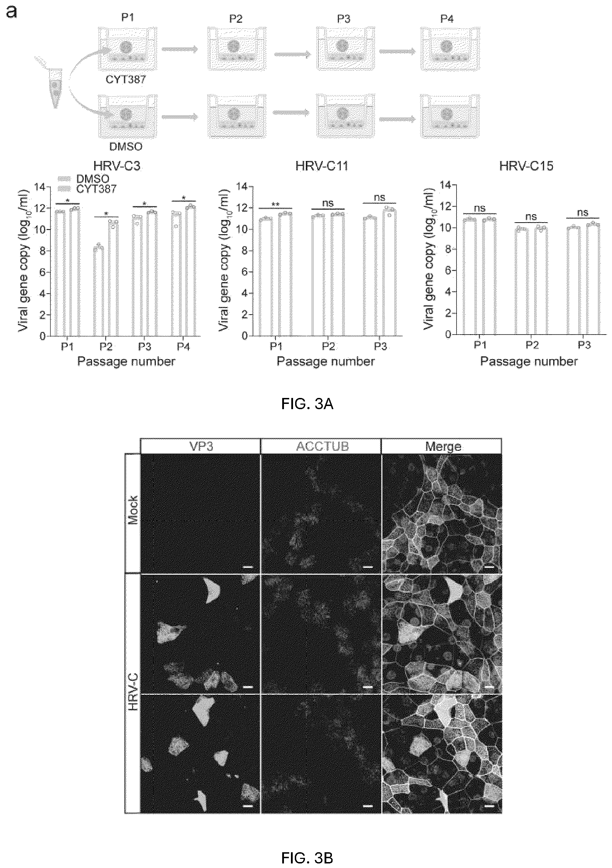

FIGs. 3A-C. Human nasal organoid sustained serial HRV-C propagation. (A) A schematic graph outlines the experimental procedure for panels b, c, and d. Nasal organoids pre-treated with CYT387 or DMSO were inoculated with HRV-C3, C11, and C15 at 100 viral gene copy/cell and incubated in the presence of CYT387 or DMSO respectively (n=3). CYT387- and DMSO-treated media were brought forward as inoculum to the next round of infection, during which CYT387 and DMSO treatment continued. Culture media were harvested at 96 h.p.i. to detect viral replication. (B) At 24 h.p.i., nasal organoids inoculated with HRV-C3 were fixed and doubled stained with an α-VP3 (green) and α-ACCTUB (red). Nuclei and actin filaments were counterstained with DAPI (blue) and Phalloidin-647 (white), respectively. Scale bar, 10 µm. (C) TEM images of HRV-C3 viral particles in the culture media of infected nasal organoids. Arrows indicate viral particles (black) or empty capsids (white). Scale bar, 100 nm. Data represent mean and SD of the indicated number (n) of biological replicates from a representative experiment. Statistical significance (in A) was determined using a two-tailed Student's t-test. *P < 0.05, **P < 0.01. *ns* not significant. The experiments in B and C were independently performed three times with similar results.

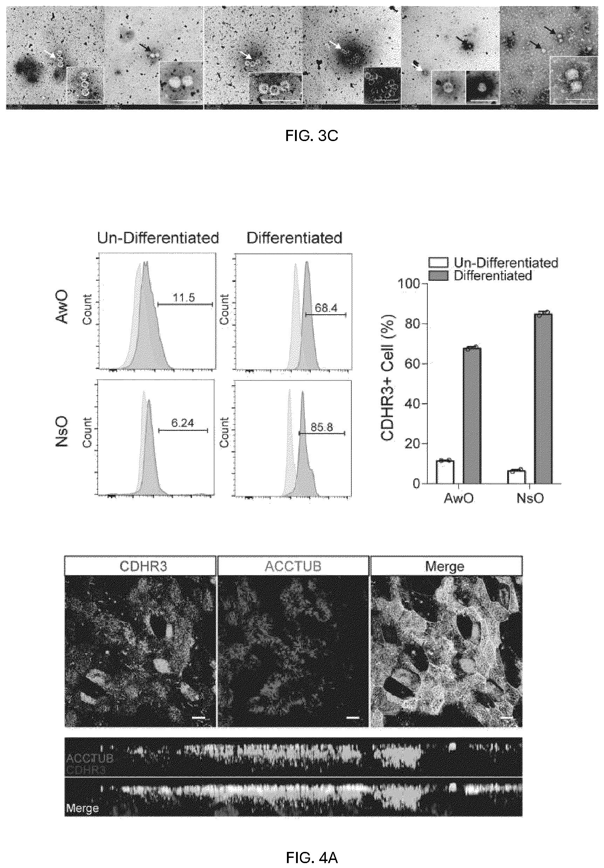

FIGs 4A-C. Receptor blocking, antiviral inhibition, and virus titration in nasal organoids. (A) Airway (AwO) and nasal organoids (NsO) and their un-differentiated counterparts were immune-stained with an α-CDHR3 and an isotype IgG control and applied to flow cytometry analysis (n=2). The left panel shows the representative histograms. Nasal organoids were fixed and double-stained with an α-CDHR3 (green), α-ACCTUB (red). Confocal images of en face (top) and cross-section (bottom) are shown. Nuclei and actin filaments were counterstained with DAPI (blue) and Phalloidin-647 (white), respectively. Scale bar, 10 µm. (B) Nasal organoids were treated with two α-CDHR3 of 20µg/ml and autologous IgG for 2 hours (n=3), followed by HRV-C3 inoculation and further incubation with the same antibodies. Culture media were harvested at the indicated h.p.i. to detect viral replication. Results show the relative viral load in the α-CDHR3 treated organoids versus those in IgG-treated organoids. Airway organoids infected with HRV-A1 or HRV-C3 were treated with 1 µM Rupintrivir or 3 µM Itraconazole or DMSO (n=3). Culture media were harvested from the infected organoids at the indicated h.p.i. and applied to the detection of viral replication by RT-qPCR. Results show the relative viral load in the drug-treated organoids versus those in DMSO-treated organoids. (C) Nasal organoids were infected with HRV-C3 after 10-fold serial dilutions (10⁻¹~10⁻³). At 24 h.p.i., the organoid monolayers were fixed and immune-stained with an α-VP3, followed by imaging analysis. Representative high-content confocal images show the VP3+ cells (green) in the monolayers infected by serially-diluted viruses. Nuclei were stained with DAPI (blue). Scale bar, 100 µm. Three different areas in each organoid monolayer were randomly selected for calculating VP3+ cells. The results show the average number of VP3+ cells at different dilutions. Data represent mean and SD of the indicated number (n) of biological replicates from a representative experiment. Statistical significance (in B) was determined using a two-tailed Student's t-test. *P < 0.05, **P < 0.01, ***P < 0.001. *ns* not significant. The experiments in A and C were performed three times independently with similar results.

### 4.1 DEFINITIONS

As used herein, the term "infection" refers to any state in at least one cell of an organism (i.e., a subject) is infected by an infectious agent. As used herein, the term "infectious agent" refers to a foreign biological entity, i.e. a pathogen. Infectious agents include, but are not limited to bacteria, viruses, protozoans, and fungi. Infectious diseases are disorders caused by infectious agents. Some infectious agents cause no recognizable symptoms or disease under certain conditions but have the potential to cause symptoms or disease under changed conditions.

The term "culture" or "culture system" means the maintenance of cells in an artificial, in vitro environment. It is used to encompass the cultivation not only of individual cells, but also of tissues or organs. The culture or culture system is under culture conditions in which the cells or tissues are grown that promote prolonged tissue expansion with proliferation, multilineage differentiation and recapitulation of cellular and tissue ultrastructure.

As used herein, the term "subject" refers to individuals (e.g., human, animal, or other organism) to be treated by the methods or compositions of the present disclosure. Subjects include, but are not limited to, mammals (e.g., murines, simians, equines, bovines, porcines, canines, felines, and the like), and includes humans. The term "subject" generally refers to an individual who will receive or who has received the medical device of the present disclosure for treatment (e.g., coated with a probiotic microbe, and optionally one or more other agents) for a condition characterized by the presence of pathogenic bacteria, or in anticipation of possible exposure to pathogenic bacteria. The term subject can be "non-human animals" including, but are not limited to, vertebrates such as rodents, non-human primates, ovines, bovines, ruminants, lagomorphs, porcines, caprines, equines, canines, felines, ayes, etc.

As used herein the term "pharmaceutical composition" refers to pharmaceutically acceptable compositions, wherein the composition comprises a pharmaceutically active agent, and in some embodiments further comprises a pharmaceutically acceptable carrier. In some embodiments, the pharmaceutical composition may be a combination of pharmaceutically active agents and carriers.

As used herein, the terms "treatment," "treating," and the like, refer to administering an agent, or carrying out a procedure, for the purposes of obtaining an effect on or in a subject, individual, or patient. The effect may be prophylactic in terms of completely or partially preventing a disease or symptom thereof and/or may be therapeutic in terms of effecting a partial or complete cure for a disease and/or symptoms of the disease. "Treatment," as used herein, may include treatment of infection in a mammal, particularly in a human, and includes one or more of: (a) preventing disease; (b) inhibiting the disease, i.e., arresting its development; and (c) relieving the disease or its symptoms, i.e., causing regression of the disease or its symptoms. Treating may also refer to any indicia of success in the treatment or amelioration or prevention of a disease, including any objective or subjective parameter such as abatement; remission; diminishing of symptoms or making the disease condition more tolerable to the patient; slowing in the rate of degeneration or decline; or making the final point of degeneration less debilitating. The treatment or amelioration of symptoms can be based on objective or subjective parameters; including the results of an examination by a physician. The term "therapeutic effect" refers to the reduction, elimination, or prevention of the disease, symptoms of the disease, or side effects of the disease in the subject.

As used herein the term "pharmaceutically acceptable" means approved by a regulatory agency of the Federal or a state government or listed in the U.S. Pharmacopoeia, other generally recognized pharmacopoeia in addition to other formulations that are safe for use in animals, and more particularly in humans and/or non-human mammals.

As used herein, the term "effective amount" refers to the amount of a composition (e.g., a probiotic microbe) sufficient to effect beneficial or desired results. An effective amount can be administered in one or more administrations, applications or dosages and is not intended to be limited to a particular formulation or administration route. It is within the skilled artisan's ability to relatively easily determine an effective amount of a probiotic microbe or other therapeutic composition.

### 5. DETAILED DESCRIPTION

In the following description, details of the present invention are set forth as preferred embodiments. It will be apparent to those skilled in the art that modifications, including additions and/or substitutions may be made without departing from the scope and spirit of the invention. Specific details may be omitted so as not to obscure the invention; however, the disclosure is written to enable one skilled in the art to practice the teachings herein without undue experimentation.

### 5.1 Respiratory Organoids

Respiratory Organoids that are used in the present disclosure refers to a three-dimensional cell aggregate and may comprise an organoid or cell cluster formed from suspension cell culture. The organoid may also be called a small organ-like organ, an organ analog, or an organ-like organ. The organoid specifically comprises one or more cell types among several types of cells constituting an organ or tissue, and are able to reproduce the form and function of the tissue or organ. The organoids recapitulate the cellular architecture and ultrastructure of the sample from which they were derived. In addition, by reaggregating and recombining cells using 3D culture to make them similar to the living environment, the limitations of 2D cell lines cultured using 2D culture can be overcome and the physiological functions of living organisms can be similarly reproduced, which can be applied to disease modeling, drug screening, etc.

The respiratory organoid that are useful in the present disclosure is an artificial organ that exhibits the function of, for example, the respiratory tract (=airway). In one embodiment, the respiratory organoids may be one of alveolar organoids and airway organoids, nasal organoids, without being limited thereto. In one embodiment, the organoids are adult stem cell-derived organoids. In one embodiment, the organoids are derived from epithelial stem cells. In one embodiment, the organoids are airway organoids. In one embodiment, the airway organoids are derived upon induction of proximal differentiation from organoids. In one embodiment, the organoids were derived from primary lung tissues.

In certain embodiments, the organoids may be cultured until they reach a size of 115 µm (±6.4), without being limited thereto. For examples, the size of the organoids may be 100 µm to 130 µm, 100 µm to 128 µm, 100 µm to 126 µm, 100 µm to 125 µm, 100 µm to 124 µm, 100 µm to 121.4 µm, 103 µm to 130 µm, 103 µm to 128 µm, 103 µm to 126 µm, 103 µm to 125 µm, 103 µm to 124 µm, 103 µm to 121.4 µm, 106 µm to 130 µm, 106 µm to 128 µm, 106 µm to 126 µm, 106 µm to 125 µm, 106 µm to 124 µm, 106 µm to 121.4 µm, 108.9 µm to 130 µm, 108.9 µm to 128 µm, 108.9 µm to 126 µm, 108.9 µm to 125 µm, 108.9 µm to 124 µm, or 108.9 µm to 121.4 µm, without being limited thereto.

Organoids prepared by the subject methods may be used in basic research, e.g. to better understand the basis of disease and infection, and in drug discovery, e.g. as reagents in screens such as those described further below, and for diagnostic purposes. Organoids are also useful for assessing the pharmacokinetics and pharmacodynamics of an agent, e.g. the ability of a mammalian tissue to absorb an active agent, the cytotoxicity of agents on primary mammalian tissue, etc. The immune component of these organoids are useful for assessing vaccines, adjuvants, or identifying the effects of candidate therapeutics on disease or infection-related immune responses.

### 5.2 Cultures

### 5.2.1 Organoids Culture and composition

Culture systems, compositions and methods are provided for culture of adult stem cell-derived organoids. The cultures can be maintained for up to 5 days, up to 7 days, up to 10 days, up to 15 days, up to 21 days, up to 28 days, up to 100 days, up to 180 days, over 6 months or over a year. In some embodiments, tissue or stem cells are obtained from any mammalian species, e.g. human, equine, bovine, porcine, canine, feline, rodent, e.g. mice, rats, hamster, primate, etc.

In one embodiment, respiratory tissue may be obtained by any convenient method, e.g. by biopsy, during surgery, by needle, etc., and is typically obtained as aseptically as possible. Nasal epithelial cells are procured invasively using a nasal swab from the nasal cavity of an individual. In one embodiment, suitable culture medium for the respiratory organoids is known in the art and also disclosed in Section 6.6.1 of the present disclosure.

The organoid cultures are used for modeling respiratory pathologies, including, for example, infection, autoimmunity, cancer, etc.; and for screening therapeutics to treat such pathologies; for precision medicine; and stem cell-based therapies. Screening assays useful to determine complex responses to therapies, including but not limited to antiviral therapies, anti-inflammatory therapies, immune therapies, and the like are provided.

### 5.2.2 Infection and Growth Culture

In one embodiment, suitable culture medium for the respiratory organoids to promote infection and growth of respiratory microorganisms is disclosed in Section 6.6.1 of the present disclosure. In one embodiment, the organoid medium includes one or more drug for the treatment of myelofibrosis, inhibitors of interferon and interferon-stimulated gene expression through JAK1/JAK2 (kinases mediating IFN signaling) and TBK1/IKKε (kinases mediating RLR and TLR signaling) pathways. In certain embodiments, the organoid medium comprises CYT387, Amlexanox, BX795, MRT67307, Ruxolitinib or a combination thereof. In one embodiment, the organoid medium comprises CYT387.

In one embodiment, the organoids prepared by the disclosed methods may be used propagate a respiratory microorganism. In one embodiment, the presently disclosed methods sustain serial passage of the microorganisms. In one embodiment, the presently disclosed methods may be used to propagate and sustain passage of respiratory microorganisms that are uncultivable respiratory microorganisms.

### 5.3 Respiratory Microorganisms

Respiratory microorganisms include viral, bacterial, and fungal infectious agents. Respiratory microorganisms include respiratory pathogens, e.g. viruses, bacteria and protozooans that infect the lungs, particularly in lower respiratory infections. Pathogens of interest include, for example, influenza, rhinovirus, adenovirus, coronavirus, including but not limited to, SARS-CoV1, SARS-CoV2, MERS-CoV; tuberculosis. Respiratory viruses cause many diseases in humans, with significant impact on morbidity and mortality worldwide, mainly in children. A number of human respiratory viruses circulate commonly in all age groups and are recognized as adapted to efficient person-to-person transmission. Respiratory pathogen can be used to infect a respiratory organoid culture, the pathogen can be propagated and studied by monitoring and screening methods for compounds that are useful to treat respiratory diseases. Common respiratory viruses include but are not limited to, influenza: ADV, adenovirus; HBoV, human bocavirus; HCoV, human coronavirus including SARS-CoV1 and SARS-CoV2; HMPV, human metapneumovirus; HPIV, human parainfluenza virus; HRSV, human respiratory syncytial virus; HRV, human rhinovirus.

Influenza virus. There are four types of influenza viruses, A, B, C, and D. Influenza types A and B cause human infection annually during the epidemic season. Influenza A has several subtypes according to the combination of hemagglutinin (H) and the neuraminidase (N) proteins that are expressed on the surface of the viruses. Influenza viruses have a negative-sense, single-stranded RNA genome that is segmented. There are 18 different hemagglutinin subtypes and 11 different neuraminidase subtypes (H1-18 and N1-11). Influenza A viruses can be characterized by the H and N types such as H1N1 and H3N2. Influenza B viruses are classified into lineages and strains. Influenza viruses replicate in the epithelial cell lining of the upper and lower respiratory tracts. Illness during infection is primarily the result of lung inflammation and compromise caused by epithelial cell infection and death, combined with inflammation caused by the immune system's response to infection. Severe respiratory illness can be caused by multiple, non-exclusive mechanisms, including obstruction of the airways, loss of alveolar structure, loss of lung epithelial integrity due to epithelial cell infection and death, and degradation of the extracellular matrix that maintains lung structure. In particular, alveolar cell infection appears to drive severe symptoms since this results in impaired gas exchange and enables viruses to infect endothelial cells, which produce large quantities of pro-inflammatory cytokines. Pneumonia caused by influenza viruses is characterized by high levels of viral replication in the lower respiratory tract, accompanied by a strong pro-inflammatory response

Human respiratory syncytial virus (HRSV) virions are heterogeneous in size and shape, and consist of a helical nucleocapsid containing a negative-sense single-stranded RNA, tightly bound to the nucleoprotein (N). Two HRSV groups, A and B, were originally distinguished based on the antigenic differences in the attachment glycoprotein G. HRSV is the single most frequent cause of lower respiratory tract infection (LRTI) leading to morbidity and mortality in children worldwide. HRSV replicates in respiratory epithelia, reaching high titers in nasal secretions and causing virus shedding for up to 3 weeks after the end of symptoms. Cell-to-cell spread leads to involvement of the entire respiratory tree by HRSV, reaching bronchioles 1-3 days after the onset of symptoms, inducing necrosis of ciliated cells, syncytia formation, peribronchiolar inflammation with abundant lymphocytes and macrophages, and impairment of secretion clearance, resulting in small airway obstruction and lung hyperinflation typical of bronchiolitis. HRSV pneumonia with interstitial mononuclear infiltrate, eosinophilic cytoplasmic inclusions in epithelial cells, and multinucleated giant cells, frequently coexist with bronchiolitis. HRSV disease is particularly severe in young babies, whose immature airways are unable to compensate for the virus-induced damage.

Human parainfluenza viruses (HPIVs) are frequent causes of LRTIs in infants and children worldwide. HPIVs share structural and biological characteristics with HRSV and are distributed in two genera of the family Paramyxoviridae. HPIVs are classified antigenically into types 1-4, and HPIV-4 has subtypes A and B. The virions are pleomorphic, with single-stranded negative-sense RNA, ranging from 150 to 200 nm in diameter. The virus does not persist long in the environment and is transmitted mainly by large droplets and fomites. HPIV replicates in ciliated cells causing cytolysis of the respiratory mucosa. The infection begins in the upper respiratory tract and disseminates down the respiratory tree. The larynx and trachea are mostly involved in the croup syndrome, and extensive involvement of the lower respiratory tree may be present in tracheobronchitis, bronchopneumonia, and bronchiolitis. Similar to what occurs with HRSV, amplified inflammatory response induced by viral infection of epithelial cells causes mononuclear interstitial infiltrate, epithelial necrosis, inflammatory exudate into the alveoli, and hyaline membrane formation in the lungs. In cases of croup, mononuclear inflammatory cell infiltrate is seen in the subglotic area.

Human metapneumovirus (HMPV) is a frequent cause of community-acquired ARI in children and adults worldwide. HMPV particles are enveloped, pleomorphic, spherical, or filamentous particles, of about 209 nm in diameter. Like other paramyxoviruses, HMPV has a negative-sense, single-stranded RNA genome, and the viral replication occurs in a gradient manner. Little is known about HMPV-specific mechanisms of pathogenesis. Animal studies show disruption of the respiratory epithelium, epithelial cell sloughing, and inflammatory infiltrates in the lung. In pathologic studies of humans with underlying diseases and HMPV infection, the main findings are acute and organizing lung injury, diffuse alveolar damage, sloughed epithelial cells with eosinophilic cytoplasmic inclusions, multinucleated giant cells, histiocytes, and hyaline membrane formation.

Human rhinoviruses (HRVs) are the most frequent respiratory pathogens of humans, and the most commonly detected viruses in samples from common cold sufferers. HRVs are small, nonenveloped, positive-stranded RNA viruses in the family Picornaviridae, genus Rhinovirus, distributed in two species, A (75 serotypes) and B (25 serotypes). HRV replication is restricted to the respiratory epithelium, taking place in scattered ciliated cells of the nose and in nonciliated cells of the nasopharynx, and this tropism seems to be a consequence of receptor availability. Infection of a limited number of cells triggers the nuclear translocation of NF-κB and gene expression of cytokines, chemokines, and inflammatory mediators. These, in association with the stimulation of local parasympathetic nerve endings, result in the development of cold symptoms. Kinins, prostaglandins, proinflammatory cytokines, and chemokines may contribute to vasodilation, increased vascular permeability, influx of polymorphonuclear leukocytes, exocrine gland secretion, and nerve ending stimulation, resulting in nasal obstruction, rhinorrhea, sneezing, cough, and sore throat.

Adenoviruses are nonenveloped, icosahedral DNA viruses of the genus Mastadenovirus, family Adenoviridae. Respiratory infections by adenoviruses occur worldwide and with no apparent seasonality. Symptomatic infections may involve all parts of the respiratory tract and generally initiate in the upper respiratory epithelium. Adenovirus infection results in necrosis of cells of airway epithelia and may cause viremia by systemic virus dissemination in immunocompromised persons. Bronchiolitis, interstitial pneumonitis, and mononuclear cell infiltrates are part of the inflammatory process in the lungs. In addition to lytic infection, adenoviruses may become latent in epithelial and lymphoid cells, which is probably important to maintain the virus in populations.

Human coronavirus (HCoV) subtypes 229E and OC43 were the only coronaviruses identified in humans until 2003, when identification of the severe acute respiratory syndrome (SARS) coronavirus led to a renewed research on HCoVs. Coronaviruses are enveloped viruses with distinct virion morphology, displaying widely spaced, long petal-shaped spikes at the surface, that confer to the virus a crownlike appearance, origin of the name corona. The viral envelope contains a long helical nucleocapsid with single-, positive-stranded. HCoVs have been found throughout the world and are considered to be the second most frequent cause of common colds. HCoV-229E and -OC43 cause common colds with variable frequency.

SARS-CoV-2 is a virus of the species severe acute respiratory syndrome-related coronavirus (SARSr-CoV), related to the SARS-CoV-1 virus. It is of zoonotic origins and has close genetic similarity to bat coronaviruses. SARS-CoV-2 is a member of the subgenus Sarbecovirus (beta-CoV lineage B). Coronaviruses undergo frequent recombination. There are many thousands of variants of SARS-CoV-2, which can be grouped into the much larger clades.

Human bocavirus (HBoV) is a novel member of the family Parvoviridae, provisionally classified by sequence homology and genome organization in the genus Bocavirus, which already included two other viruses: the bovine parvovirus 1 and canine minute virus. HBoV virions consist of small nonenveloped icosahedral particles with a single-stranded DNA genome of approximately 5300 nt, organized in a way similar to that of other known bocaviruses, with three ORFs, two encoding the nonstructural proteins NS1 and NP-1, and a third nesting the two capsid proteins, VP1 and VP2. HBoV circulates worldwide and prevalence studies, done mostly by PCR in respiratory samples from children, have shown detection rates of 1.5-19%, depending on the regions where studies were conducted and the sensitivity of the assays. The sites of HBoV replication have not been determined, but the virus is apparently not confined to the respiratory tract, since it has been detected in stools and serum, suggesting systemic infection.

Bacterial and protozoan pathogens are less common than viral but can include Mycobacterium tuberculosis, Streptococcus pneumoniae, Mycoplasma pneumoniae, Hemophilus influenzae, Chlamydophila pneumoniae; Chlamydia psittaci; Coxiella burnetiid; Legionella pneumophila Staphylococcus aureus; Klebsiella pneumoniae; cryptosporidiosis can cause outbreaks and sporadic cases of respiratory illness.

In one aspect, the transmission and pathogenesis of respiratory pathogens can be studied using the respiratory organoids which can serve as a highly physiological relevant model to study the tropism and pathogenesis of respiratory pathogens.

### 5.4 Screening of Compound

In one aspect, the disclosure provides a method of identifying a therapeutic agent effective for treating a respiratory disease. This method includes providing a culture composition, infecting the culture composition with a respiratory pathogen, administering the therapeutic agent to the culture composition, and determining whether the therapeutic agent is effective in treating or preventing the respiratory disease caused by the infection in the respiratory organoids. The therapeutic agent may be administered to the culture composition before or after the culture composition is infected with the respiratory pathogen.

Screening is performed using respiratory infectious pathogens, e.g. bacteria, viruses, and the like. In one aspect, a method is provided for in vitro screening for agents for their effect on cells of different cell types, e.g. epithelial, immune, stromal present in an organoid, including processes of viral infection initiation and treatment. Organoids cultured by the methods described herein are exposed to candidate agents, optionally before or after exposure to a respiratory pathogen. Agents of interest include pharmaceutical agents, e.g. known therapeutic agents; small molecules, antibodies, peptides, etc., and genetic agents, e.g. antisense, RNAi, expressible coding sequences, and the like, e.g. expressible coding sequences for candidate secreted growth factors, cytokines, receptors or inhibitors thereof, or other proteins of interest, and the like. In some embodiments the effect of candidate therapeutic agents on viral infection-related immune responses or their downstream effects is determined, for example where agents may include, without limitation, chemotherapy, monoclonal antibodies or other protein-based agents, radiation/radiation sensitizers, cDNA, siRNA, shRNA, small molecules, and the like. Methods are also provided for using the organoid culture to screen for agents that modulate tissue function.

### 5.5 Pharmaceutical Composition

Once a compound is identified to be useful in affecting the respiratory organoids or the pathogens that infected the respiratory organoids using the screening method of the present disclosure, a pharmaceutical composition comprising the identified compound may be prepared. A pharmaceutical composition according to the present disclosure may be formulated for external application such as powders, granules, tablets, capsules, suspensions, emulsions, syrups, or aerosols or in the form of a sterile injectable solution, examples of a carrier, excipient and diluent that can be comprised in the composition comprise lactose, dextrose, sucrose, oligosaccharide, sorbitol, mannitol, xylitol, erythritol, maltitol, starches, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In the case of the formulations, the formulation may be prepared by using a diluent or an excipient, such as a filler, an extender, a binder, a wetting agent, a disintegrating agent, and a surfactant which are generally used. Solid formulations for oral administration comprise tablets, pills, powders, granules, capsules, and the like, and the solid formulation may be prepared by mixing at least one excipient, for example, starch, calcium carbonate, sucrose or lactose, gelatin, and the like. In addition, lubricants such as magnesium stearate, talc may be used in addition to simple excipients. Liquid formulations for oral administration may correspond to suspensions, oral liquids, emulsions, syrups, and the like, and may comprise various excipients, for example, a wetting agent, a sweetener, an aromatic agent, a preservative, and the like, in addition to water and liquid paraffin which are commonly used as simple diluents. Formulations for parenteral administration comprise sterilized aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used.

The pharmaceutical composition of the present disclosure can be administered orally or parenterally, preferably parenterally, and in the case of parenteral administration, it may be administered by intramuscular injection, intravenous injection, subcutaneous injection, intraperitoneal injection, topical administration, transdermal administration, etc.

The appropriate dosage of the pharmaceutical composition of the present disclosure may vary depending on factors such as formulation method, administration method, patient's age, weight, sex, pathological condition, diet, administration time, administration route, excretion rate and reaction sensitivity.

The pharmaceutical composition of the present disclosure may be formulated in unit dosage form using a pharmaceutically acceptable carrier and/or excipient according to a method that can be easily performed by a person skilled in the art to which the disclosure pertains, or may be manufactured by placing it in a bulk container. Here, the formulation may be in the form of a solution, suspension or emulsion in an oil or aqueous medium, or may be in the form of an extract, powder, granule, tablet or capsule, and may additionally comprise a dispersant or a stabilizer.

### 5.6 Methods of Treatment

A respiratory disease that can be treated using the method of the present disclosure is a physically manifest disease or condition related to the respiratory system, and is, for example, cystic fibrosis, respiratory distress syndrome, acute respiratory distress syndrome, pulmonary tuberculosis, cough, bronchial asthma, cough based on increased airway hyperreactivity (bronchitis, influenza syndrome, asthma, obstructive pulmonary disease, etc.), flu syndrome, cough suppression, airway hyperresponsiveness, tuberculosis disease, asthma (airway inflammatory cell infiltration, increased airway hyperresponsiveness, bronchoconstriction, mucus hypersecretion, etc.), chronic obstructive pulmonary disease, emphysema, pulmonary fibrosis, idiopathic pulmonary fibrosis, reversible airway obstruction, adult respiratory disease syndrome, pigeon breeder's disease, farmer's lung, bronchopulmonary dysplasia, airway disease, emphysema, allergic bronchopulmonary aspergillus, allergic bronchitis bronchiectasis, occupational asthma, reactive airway disease syndrome, interstitial lung disease, parasitic lung disease, etc., without being limited thereto.

### 5.7 Kits

The present disclosure provides a kit for producing respiratory organoids for propagating microorganisms, comprising (a) a composition for producing respiratory organoids in a culture medium comprising a JAKL1/JAK2 inhibitor or TBK1/IKKε inhibitor; and (b) a respiratory microorganism for infecting the respiratory organoids, wherein the microorganism propagates in the respiratory organoids.

The kit of the present disclosure may further comprise an instruction. The instruction may comprise the production method, without being limited thereto.

In certain embodiments, the kit may comprise a container; an instruction; etc. The container can serve to package the materials, as well as store and secure them. The container may take the form of, for example, a bottle, a tub, a sachet, an envelope, a tube, an ampoule, etc., which may be partially or wholly plastic, glass, paper, foil, etc. The container may be equipped with a fully or partially separable stopper, which may initially be part of the container or attached to the container by mechanical, adhesive, or other means. The container may also be equipped with a stopper, which may be accessible to the contents by a needle. The kit may comprise an external package, which may comprise an instruction for use of the components.

### 6. EXAMPLES

### 6.1 Human airway organoids susceptible to HRV-C positive clinical specimens

A total of nine achieved HRV-C positive nasopharyngeal specimens with variable viral loads were obtained. We inoculated these specimens onto the 2D human airway organoids, monolayers of airway organoids growing on transwell inserts. We harvested an aliquot of culture medium from the top chamber on the indicated days post-inoculation to detect viral load (copy number of viral 5'UTR) over time. As shown in FIG. 1B, 8 out of 9 specimens showed an increased viral load after inoculation, indicating a productive HRV-C infection of the airway organoids. Phylogenic analysis of the 5'UTR of the eight specimens revealed that specimens 1, 4, 5, and 7 were closely related to HRV-C15, while the others were related to HRV-C3, -C8, - C11, and -C45. Phylogenetic analysis based on the VP4/VP2 genes, which are also commonly used for rhinovirus genotyping, showed similar results. However, specimen 6 was genotyped to be HRV-C45 and HRV-C11 based on the 5'UTR and VP4/VP2 sequences, respectively. The inconsistency was also reported in HRV genotyping previously²⁴. We then examined HRV-C viral growth and release in airway organoids. After the inoculation of an HRV-C3 positive (HRV-C3+) specimen, we harvested culture media from the top and bottom chambers for viral load detection. As shown in FIG. 1C, viral loads were remarkably higher in the apical media than in the basolateral media, suggesting that progeny virions were preferentially released from the apical side. HRV-A was inoculated onto the airway organoids in parallel. We observed a productive infection of HRV-A with a similar pattern of virus release (FIG. 1C).

Understanding the biology of a virus invariably starts with reproducible virus isolation and cultivating a sufficient amount of viruses, followed by a series of in vitro and in vivo characterizations of virus biology and virus-host interaction. Given that airway organoids were susceptible to HRV-C infection and sustained active viral replication, we inferred that airway organoids might sustain serial virus passage, enabling reproducible HRV-C isolation and cultivation. We were prompted to propagate the progeny virions using the culture media from the first round of infection with HRV-C+ clinical specimens. However, when the HRV-C+ media were used to inoculate a new batch of airway organoids, we did not observe any sign of viral growth. Namely, airway organoids, despite the susceptibility to HRV-C, were unable to sustain viral serial passage.

### 6.2 Immunosuppression enabling serial HRV-C passage in airway organoids

Prior studies in experimental mice and human epithelial organoids documented that mucosal epithelial cells elicited a robust antiviral response upon viral infections ²⁵⁻²⁹. We inferred that the antiviral response triggered in airway organoids by HRV-C infection might restrain serial viral passage. CYT387, a drug for the treatment of myelofibrosis, can inhibit interferon and interferon-stimulated gene expression through JAK1/JAK2 (kinases mediating IFN signaling) and TBK1/IKKε (kinases mediating RLR and TLR signaling) pathways²⁸. A clinical study reported that JAK1/JAK2 inhibition rendered patients at a higher risk of rhinovirus infection³⁰. Therefore, we hypothesized that treatment with CYT387 or other molecules may repress the virus-induced antiviral response in airway organoids, boost viral propagation, and enable serial HRV-C passage.

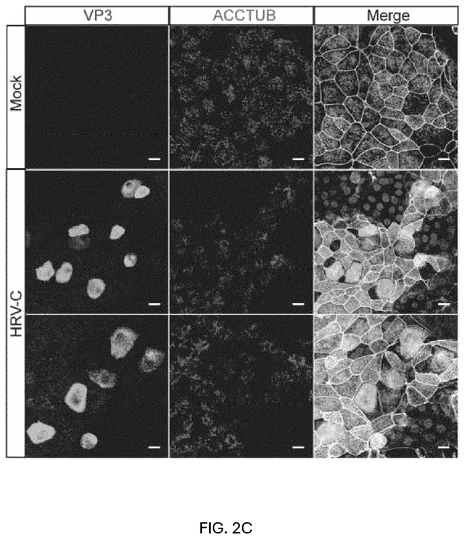

We examined HRV-C replication in airway organoids in the presence or absence of CYT387 (FIG. 2A). Briefly, we inoculated an HRV-C3+ medium (medium collected from organoids infected by an HRV-C3+ clinical specimen) onto the airway organoids pre-treated with 1 µg/ml CYT387 or DMSO. The infected organoids were further incubated with CYT387 (CYT thereafter) or DMSO for 96 hours, after which we harvested the culture medium (P1) to examine viral growth. P1 media from CYT- and mock-treated organoids were brought forward to P2 passage in the presence of CYT and DMSO, respectively (FIG. 2A). As shown in FIG. 2B, CYT treatment resulted in an elevated viral load in P1 media at 96 hours post-infection (h.p.i.). In P2, CYT-treated organoids still sustained robust viral replication, whilst no viral growth was observed in DMSO-treated organoids. In the following P3 passage, we had to inoculate CYT-treated P2 media onto airway organoids, in which we continued CYT or DMSO treatment to verify the enhancement effect of CYT. This CYT-treated media inoculation, followed by CYT or DMSO treatment, was repeated in the subsequent P4 and P5 passage. In the presence of CYT, HRV-C3 was consecutively passaged five times in airway organoids, with a higher viral load than that in DMSO-treated organoids (FIG. 2C). Moreover, HRV-C virions serially propagated in CYT-treated organoids established a productive infection in airway organoids without CYT treatment, indicating that progeny virions passaged in airway organoids in the presence of CYT were highly infectious (FIG. 2D). Overall, the results demonstrate that airway organoids enabled serial HRV-C propagation in the presence of CYT and probably other immunosuppressive molecules.

### 6.3 Nasal organoids sustaining serial HRV-C propagation

We then performed virus passage in nasal organoids, the organoid model with a cellular composition comparable to airway organoids, yet better resembles the upper respiratory epithelium¹⁷. We inoculated nasal organoids with the HRV-C3+ medium collected from organoids infected with an HRV-C3+ clinical specimen in the presence of CYT or DMSO and collected culture media at 96 h.p.i. Due to the high viral load in DMSO-treated nasal organoids in a preliminary experiment, we changed the experimental design. Briefly, the virus-containing media from CYT- and DMSO-treated organoids were brought forward to the next round of infection with the CYT and DMSO treatment, respectively (FIG. 3A). Notably, CYT treatment significantly boosted viral growth (FIG. 3B). Nonetheless, in the absence of CYT, nasal organoids derived from two different donors sustained four consecutive passages of HRV-C3 (FIG. 3B). We also tested HRV-C11+ (FIG. 3C) and HRV-C15+ (FIG. 3D) medium (the medium collected from organoids infected with an HRV-C11+ and HRV-C15+ clinical specimen respectively) in nasal organoids derived from another donor. The two subtypes of HRV-C were serially passaged three times with high viral loads, and more passages could be furthered, if necessary. Overall, nasal organoids per se sustained serial propagation of HRV-C without any intervention. To verify the infectiousness, we demonstrated that HRV-C passaged multiple times in the nasal organoids established a productive infection in nasal organoids (FIG. 3E). HRV-C virions released from the nasal organoids were concentrated and applied to transmission electron microscope (TEM) examination. Virion particles sized around 30 nm, and empty capsid particles were readily discernible (FIG. 3F). We sequenced the whole genome of the viruses in the initial clinical specimen and the viruses after 1 and 6 consecutive passages with CYT treatment; no adaptive mutation was identified. Collectively, airway and nasal organoids enabled reproducible virus isolation and serial passage of poorly cultivable HRV-C.

### 6.4 Receptor blocking, antiviral inhibition, and virus titration in nasal organoids

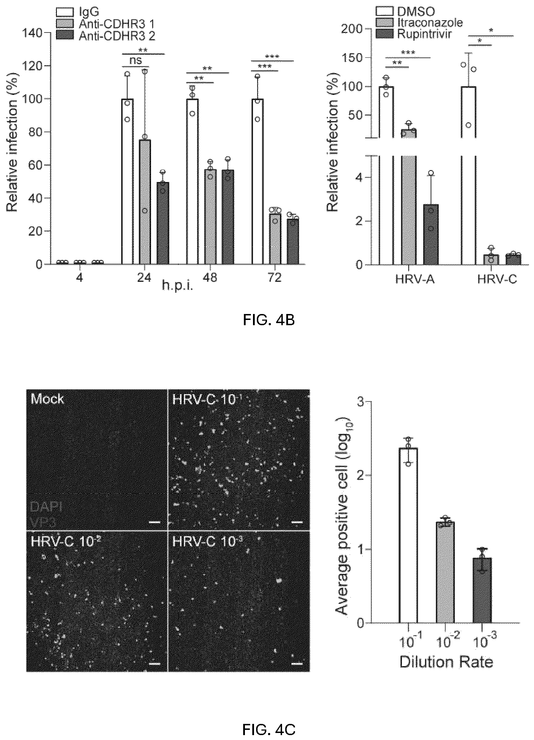

HRV infection is initiated upon virus binding to cognate receptors and receptor-mediated endocytosis. CDHR3, the cellular receptor of HRV-C6, a member of the cadherin family of transmembrane proteins, is highly expressed in human airway epithelium, especially in ciliated cells in the native airway epithelium³¹. First, we examined CDHR3 expression and distribution in the airway and nasal organoids in comparison with the undifferentiated counterparts that serve to maintain and expand the organoid culture. Flow cytometry analysis showed that the percentage of CDHR3+ cells increased from less than 20% in the undifferentiated organoids to over 60% and 80% in the differentiated airway and nasal organoids, respectively (FIG. 4A). Confocal imaging revealed high CDHR3 expression in the differentiated nasal and airway organoids (FIG. 4B); most ACCTUB+ positive ciliated cells expressed CDHR3 on the apical surface, a small portion of CDHR3+ cells were non-ciliated cells.

We then performed an antibody-blocking experiment in nasal organoids to assess the role of CDHR3 on HRV-C replication. We examined HRV-C replication in the nasal organoids in the presence of two α-CDHR3 antibodies or an isotypic IgG. Blocking CDHR3 with specific antibodies significantly decreased viral growth, verifying that CDHR3 is necessary for HRV-C entry into nasal organoids (FIG. 4C). To demonstrate the application of respiratory organoids for identifying antiviral agents against HRV-C, we tested an HRV inhibitor, Rupintrivir targeting HRV 3C protease as a proof-of-concept. Itraconazole³², a repurposed antifungal drug with documented efficacy against rhinoviruses, was also tested. We examined the effect of these two drugs against HRV-C and HRV-A in airway organoids and observed a more prominent inhibitory effect of both drugs on HRV-C than HRV-A (FIG. 4D).

Currently, few in vitro assays are available to quantify HRV-C infectious particles since no standard cell lines are susceptible to the virus, except for an adaptive strain C15a which developed a cytopathic effect and formed plaques in a CDHR3-expressing stable cell line developed by Bochkov et al³³. The quantification of HRV-C primarily relies on RT-qPCR assays to determine the viral gene copy number as we demonstrated above. Given the susceptibility of airway and nasal organoids to HRV-C, we sought to establish an organoid-based immunofluorescence assay (IFA) to quantify HRV-C infectious virions. An aliquot of HRV-C collected from the infected organoids with 1.2 × 10¹¹ viral gene copy/ml was applied to inoculate nasal organoids in triplicate after serial dilution. The organoid monolayers were fixed at 24 h.p.i. and applied to immunostaining with an α-VP3 antibody to label HRV-C infected cells, followed by analysis in a high-content imaging analysis system. FIG. 4E shows representative images of the organoids infected with serially diluted virus. A total of 3 areas were randomly selected from each organoid monolayer, from which the number of VP3⁺ cells was calculated. The average number of VP3⁺ cells was shown in FIG. 4F. We calculated the virus titer based on the image analysis of organoids inoculated with the highest dilution factor 10³; the number of infectious particles in the sample was around 3.7 × 10⁶ IFU/ml.

### 6.5 Discussion

We have established the first organoid culture system of human respiratory epithelium from primary lung tissues and nasal epithelial cells that allows for the reconstitution and expansion of the entire human respiratory epithelium in culture plates with excellent efficiency and stability ¹³⁻¹⁷. Given that nasal and airway organoids accurately simulate the native airway epithelium, we hypothesized that these respiratory organoids may sustain a productive infection of HRV-C, a common respiratory virus refractory to routine virus cultivation. Moreover, the high stability and easy availability of these respiratory organoids offered by the robust culture system may provide a reproducible cultivation system for HRV-C, eliminating a long-standing obstacle to understanding this common human respiratory virus. Indeed, after inoculation of HRV-C⁺ clinical specimens, we observed an active viral growth, indicating that airway organoids can recapitulate the susceptibility of the human airway epithelium to HRV-C (FIG. 1B). However, serial HRV-C propagation in airway organoids was unsuccessful unless an immuno-suppressive molecule CYT387 was applied to attenuate the antiviral response triggered in the infected organoids (FIG. 2B and 2C).

After the first demonstration of HRV-C infection in surgically resected sinus mucosa and nasal polyps4, Bochkov et al identified the cellular receptor CDHR3 for HRV-C6. The landmark discovery was followed by the establishment of a stable Hela-E8 cell line carrying a higher cell-surface CDHR3 expression variant. Through serial passage in the Hela-E8 CDHR3 cells, one HRV-C15a strain with enhanced fitness was obtained due to adaptive mutations³³. However, the two adaptive mutations only partially conferred the enhancing phenotype to other recombinant HRV-C subtypes. Thus far, this C15a appeared to be the only strain that can be propagated to a yield adequate for experimentations. Moreover, the adaptive mutations were cell-type specific; HRV-C15a virus propagated in Hela-E8 CDHR3 cells appeared to replicate less actively than wildtype C15 virus in human primary bronchial epithelial cells³³, which may hinder applications of the adaptive virus strain for understanding human HRV-C respiratory infections. In this study, we randomly selected three HRV-C+ clinical specimens with different subtypes, all of which were serially passaged in nasal organoids with excellent efficiency and notable viral genome stability (FIG. 3B, 3C, 3D). Thus, we have established a robust cultivation system to reproductively propagate the poorly cultivable HRV-C.

We observed abundant expression of HRV-C receptor CDHR3 on the apical surface of the airway and nasal organoids, particularly on the apical surface of ciliated cells (FIG. 4A, 4B,), consistent with the result of a single-cell RNA sequencing study of native human respiratory epithelial cells³¹. Blocking CDHR3 with specific antibodies significantly suppressed HRV-C viral growth (FIG. 4C). We also demonstrated the effectiveness of two antiviral drugs against HRV as a proof-of-concept (FIG. 4D). Furthermore, we developed an organoid-based IFA to quantify HRV-C infectious particles (FIG. 4E, 4F). Collectively, our studies showcase the unique strength of respiratory organoids for studying HRV-C, from reproducible propagation of the poorly cultivable virus to detailed dissection of virus-host interaction and developing antivirals. Given that receptor blockage with α-CDHR3 suppressed viral growth, an organoid-based neutralization assay can be developed to evaluate the neutralizing activity of vaccination sera and engineered antibodies against HRV-C. More importantly, our study establishes a new paradigm for propagating and studying other uncultivable human and animal viruses.

### 6.6 Materials and methods

### 6.6.1. Establishment, maintenance, and differentiation of respiratory organoids

This study was approved by the Institutional Review Board of the University of Hong Kong/Hospital Authority Hong Kong West Cluster (UW13-364 and UW21-695). Informed consent was obtained from patients and volunteers to collect human lung tissue and nasal cells. Multiple lines of organoids were established from surgically resected human lung tissues according to our previously published protocols^{13,15,18,20}. To derive lung organoids, we used normal lung tissues adjacent to the diseased tissues, which were provided to us randomly. These lung tissues typically contained bronchioles of varying sizes, surrounded by alveolar sacs. After 1 to 2 passages, the fibroblasts and other non-epithelial elements in the initial culture gradually diminished. Subsequently, the culture of pure epithelial organoids was stably expanded in the expansion medium for over one year. Nasal organoids were derived from nasal epithelial cells noninvasively collected from healthy donors with perfect efficiency and consecutively passaged in the expansion medium for up to 6 months¹⁷. The undifferentiated lung and nasal organoids were passaged every 2 to 3 weeks with a ratio between 1:3 to 1:10, depending on whether mechanical shearing or enzymatic digestion was used to split the organoids. Proximal differentiation protocols for generating mature airway and nasal organoids were described previously^{17,20}. Mature airway and nasal organoid monolayers growing on transwell inserts were used throughout this study unless indicated otherwise.

### 6.6.2 Virus isolation, infection, and detection

A total of 9 HRV-C positive archived nasopharyngeal specimens from clinical patients were used for the study. Mature human airway and nasal organoids growing on 24-transwell inserts were washed twice with the basal medium (Advanced DMEM/F-12 (Gibco) supplemented with 1% HEPES, 1% GlutaMAX, and 1% Penicillin/Streptomycin), followed by virus inoculation with the indicated viral gene copy and subsequent incubation in the PD medium (PneumaCult-ALI medium (STEMCELL Technologies) supplemented with 10 µM DAPT and 10 µM Y27632), at 37 °C for 4 h. After incubation, the airway or nasal organoids were washed 3 times with the basal medium to remove residual inoculum. The apical and basolateral chambers were replenished with 300µl and 500µl PD medium, respectively. As for CYT387 treatment, airway and nasal organoids were pre-incubated in the PD medium with 1µg/ml CYT387 (Invivogen, inh-cy87) or DMSO overnight. After virus inoculation, PD medium supplemented with 1µg/ml CYT387 or DMSO was dispersed into apical and basolateral chambers to maintain the organoids. To assess the effect of temperature on viral growth, we infected nasal organoids with HRV-C3 and HRV-A1 at 100 viral gene copy/cell and maintained the organoids at 33°C or 37°C. To demonstrate the effect of CDHR3 for HRV-C viral replication, we pretreated organoids with two antibodies against CDHR3 (Abcam, ab121337; Sigma-Aldrich, HPA011218) of 20µg/ml and autologous IgG (Abcam, ab172730) for 2 hours, followed by HRV-C3 inoculation at 1000 viral gene copy/cell and further incubation in the presence of α-CDHR3 and IgG respectively. Culture media were harvested from the infected organoids at the indicated h.p.i. to detect viral replication. To investigate the antiviral effect of inhibitors on HRV-C replication, organoids were inoculated with HRV-A1 or HRV-C3 at 1000 viral gene copy/cell and incubated with the PD medium in the presence of 1µM Rupintrivir, 3µM Itraconazole, or DMSO. The culture media were collected at the indicated h.p.i. for viral load detection.

To examine viral replication, we harvested cell-free culture media from top chambers at the indicated hours post-infection, followed by RNA extraction using the RNAeasy Mini Kit (Qiagen) and detection of viral loads with the RT-qPCR assay targeting human rhinovirus 5' UTR gene using a QuantiNova Probe RT-PCR Kit (Qiagen) and primers listed in Supplementary Table 1.

**Supplementary Table 1.**

| **Name** | **Supplier** | **Cat. no.** | **Lot no.** | **Dilution (IF)** | **Dilution (Flow)** |
|---|---|---|---|---|---|
| Mouse IgG | abeam | ab91353 | GR3327311-3 | 1:100 | 1:100 |
| Rabbit IgG | abeam | ab172730 | GR3284310-8 | 1:100 | 1:100 |
| Rhinovirus VP3 | Invitrogen | MA5-18249 | YD3903932 | 1:100 | 1:100 |
| CDHR3 | abcam | ab121337 | 1016309-2 | 1:100 | 1:100 |
| CDHR3 | Sigma-Aldrich | HPA011218 | 000006575 | 1:100 | - |
| TUBULIN | abeam | ab179509 | GR252919-6 | 1:100 | 1:1000 |
| TUBULIN | Sigma-Aldrich | T7941 | 088M4793 | 1:100 | - |
| Goat anti-Mouse 488 | invitrogen | A-11001 | 2090562 | 1:200 | 1:300 |
| Goat anti-Mouse 594 | invitrogen | A-11005 | 1750828 | 1:200 | - |
| Goat anti-Rabbit 488 | invitrogen | A-11034 | 1885241 | 1:200 | 1:300 |
| Goat anti-Rabbit 594 | invitrogen | A-11037 | 1608397 | 1:200 | 1:300 |

Thermal cycling conditions included reverse transcription at 45°C for 10 min, initial denaturation at 95°C for 5 min, followed by 40 cycles of 95°C for 5 s, and 55°C for 30 s. All experiments with live viruses were conducted in biosafety level 2 laboratories.

### 6.6.3 Flow cytometry analysis

Airway and nasal organoids were infected or mock-infected with HRV-C at 10000 viral gene copy/cell. At 24 and 48 h.p.i, the organoids were dissociated into single cells with 10 mM EDTA (Invitrogen) at 37°C for 30-60 minutes and fixed with 4% PFA for 30 minutes at room temperature. After permeabilization with 0.1% Triton X-100 for 5 minutes at 4°C, cells were incubated with primary antibodies, α-Rhinovirus VP3 (Invitrogen, MA5-18249), α-ACCTUB (Abcam, ab179504), α-CDHR3 (Abcam, ab121337) for 1 h, followed by secondary antibodies. The immune-stained cells were re-suspended in 2% FBS/PBS and analyzed using Agilent NovoCyte Quanteon Analyzer. FlowJo software was used for data processing.

### 6.6.4 Scanning and transmission electron microscopy

Virus- and mock-infected airway organoids were fixed with 2.5% glutaraldehyde (GTA). HRV-C3 virions in culture media were concentrated by ultracentrifugation and fixed with 2.5% GTA. Sample processing was performed by the Electron Microscope Unit of the University of Hong Kong. The LEO 1530 FEG Scanning Electron Microscope and Philips CM100 Transmission Electron Microscope were used for image acquisition.

### 6.6.5 Immunofluorescence staining and confocal imaging

To identify the virus-infected cells, we stained the organoids and performed confocal imaging as described elsewhere^{21,22,26,29}. Briefly, airway and nasal organoids were infected or mock-infected with HRV-C3 at 1000 viral gene copy/cell and incubated for 24 hours. After fixation with 4% paraformaldehyde (PFA) at room temperature for 1 h, membrane inserts seeded with organoids were cut and removed from transwells, and applied to immune staining. The fixed organoid monolayers were permeabilized in 0.1% Triton-X100 at room temperature for 10 min and blocked with 3% bovine serum albumin (BSA) for 1 h. Subsequently, organoids were incubated with primary antibodies, including α-Rhinovirus VP3 (Invitrogen, MA5-18249), α-CDHR3 (Abcam, ab121337), α-ACCTUB (Sigma-Aldrich, T7941; Abcam, ab179504) at 4°C overnight, followed by secondary antibodies. Nuclei and actin filaments were counterstained with DAPI (ThermoFisher) and Phalloidin-647 (Sigma-Aldrich), respectively. The organoids were then whole mounted on a glass slide with ProLong^{™} G Sigma-Aldrich lass Antifade Mountant (Invitrogen). The confocal images were acquired using a Carl Zeiss LSM 980 confocal microscope.

### 6.6.6 Organoid-based immunofluorescence assay (IFA)

The culture media harvested from HRV-C3 infected nasal organoids, after a 10-fold serial dilution, were used to inoculate nasal organoids. At 24 h.p.i., the organoid monolayers were fixed and permeabilized with 0.1% Triton X-100 for 10 min and blocked with 3% BSA for 1 h, followed by incubation with α-Rhinovirus VP3 (Invitrogen, MA5-18249), and a secondary antibody. Nuclei were counterstained with DAPI (Thermo Fisher). The organoids were then whole-mounted on a glass slide with ProLong^{™} Glass Antifade Mountant (Invitrogen). High-content confocal images were acquired using an IN Cell Analyzer 6500HS (GE Healthcare), a laser-based line scanning high-content imaging system. Image processing was performed using the IN Carta and Image J software.

### 6.6.7 Statistical analysis

Statistical analysis was conducted using GraphPad Prism 9.0. Two-tailed Student's t-test was used to determine statistical significance. The number of replicates is indicated in figure legends. *P < 0.05, **P < 0.01, ***P <0.001.

### 7. Exemplary products, systems and methods are set out in the following items:

1. A composition for culturing a respiratory microorganism, the composition comprising: (i) respiratory organoids selected from the group consisting of airway organoids and nasal organoids; and (ii) a respiratory microorganism, wherein the respiratory microorganism sustains serial propagation in the respiratory organoids.
2. The composition of item 1, wherein the respiratory organoids are airway organoids derived from lung tissue.
3. The composition of anyone of the preceding items, wherein the respiratory organoids are nasal organoids derived from nasal epithelial cells.
4. The composition of anyone of the preceding items, wherein the respiratory microorganism sustains at least 3-6 passages with genomic stability.
5. The composition of anyone of the preceding items, further comprising an immunosuppressive compound.
6. The composition of anyone of the preceding items, wherein the immunosuppressive compound interferes with an anti-microorganism response in the respiratory organoids.
7. The composition of anyone of the preceding items, wherein the immunosuppressive compound is a JAK1/JAK2 inhibitor or a TBK1/IKKε inhibitor.
8. The composition of anyone of the preceding items, wherein the immunosuppressive compound is Momelotinib (CYT387).
9. The composition of anyone of the preceding items, wherein the respiratory microorganism is a respiratory virus or a respiratory bacteria.
10. The composition of anyone of the preceding items, wherein the respiratory virus is HRV-C, HRV-C3, HRV-C8, HRV-C11, HRV-C15, HRV-C45, influenza virus, adenovirus, human bocavirus, human coronavirus including SARS-CoV1 and SARS-CoV2, human metapneumovirus, human parainfluenza virus, human respiratory syncytial virus, or human rhinovirus.
11. The composition of anyone of the preceding items, wherein the respiratory bacteria is *Mycobacterium tuberculosis, Streptococcus pneumoniae, Mycoplasma pneumoniae, Hemophilus influenzae, Chlamydophila pneumoniae, Chlamydia psittaci, Coxiella burnetiid, Legionella pneumophila, Staphylococcus aureus,* or *Klebsiella pneumoniae.*
12. The composition of anyone of the preceding items, wherein the respiratory organoids are human respiratory organoids comprising human respiratory epithelial cells.
13. The composition of anyone of the preceding items, wherein the respiratory organoids comprise human lung epithelial cells.
14. The composition of anyone of the preceding items, wherein the respiratory organoids comprise human nasal epithelial cells.
15. The composition of anyone of the preceding items, wherein the respiratory organoids are derived from human epithelial stem cells.
16. The composition of anyone of the preceding items, wherein the respiratory organoids are viable in culture for a period of at least 30 days.
17. A method for culturing respiratory microorganisms comprising: (i) preparing respiratory organoids, wherein the respiratory organoids are selected from the group consisting of airway organoids and nasal organoids; and (ii) infecting the respiratory organoids with a microorganism, wherein the microorganism sustains propagation in the respiratory organoid.
18. The method of item 17, further comprising a step of isolating and cultivating the respiratory microorganisms.
19. The method of anyone of the preceding items, wherein the respiratory organoids are airway organoids derived from lung tissue.
20. The method of anyone of the preceding items wherein the respiratory organoids are nasal organoids derived from nasal epithelial cells.
21. The method of anyone of the preceding items, wherein the respiratory organoids are prepared by (a) providing a fragment of an airway tissue or nasal tissue; and (b) culturing the fragment of airway or nasal tissue in a culture medium for a period of time sufficient to form the respiratory organoids.
22. The method of anyone of the preceding items, wherein the culture medium further comprises an immunosuppressive compound.
23. The method of anyone of the preceding items, wherein the immunosuppressive compound interferes with an anti-microorganism response in the respiratory organoids.
24. The method of anyone of the preceding items, wherein the immunosuppressive compound is a JAK1/JAK2 inhibitor or a TBK1/IKKε inhibitor.
25. The method of anyone of the preceding items, wherein the immunosuppressive compound is Momelotinib (CYT387), Amlexanox, BX795, MRT67307, Ruxolitinib or a combination thereof.
26. The method of anyone of the preceding items, wherein the respiratory microorganism sustains at least 3-6 passages with genomic stability.
27. The method of anyone of the preceding items, wherein the respiratory microorganism is a respiratory virus or a respiratory bacteria.
28. The method anyone of the preceding items, wherein the respiratory virus is HRV-C, HRV-C3, HRV-C8, HRV-C11, HRV-C15, HRV-C45, influenza virus; adenovirus; human bocavirus; human coronavirus including SARS-CoV1 and SARS-CoV2; human metapneumovirus; human parainfluenza virus; human respiratory syncytial virus; and/or human rhinovirusor a combination thereof.
29. The method of anyone of the preceding items, wherein the respiratory bacteria is *Mycobacterium tuberculosis, Streptococcus pneumoniae, Mycoplasma pneumoniae, Haemophilus influenzae, Chlamydophila pneumoniae; Chlamydia psittaci; Coxiella burnetiid; Legionella pneumophila, Staphylococcus aureus;* and/or *Klebsiella pneumoniae.*
30. The method of anyone of the preceding items, wherein the respiratory organoids are human respiratory organoids comprising human respiratory epithelial cells.
31. The method of anyone of the preceding items, wherein the respiratory organoids are viable in culture for a period of at least 30 days.
32. A system for culturing respiratory microorganisms comprising: (i) a fragment of lung tissue in a culture medium comprising a JAK1/JAK2 inhibitor or a TBK1/IKKε inhibitor cultured for a period of time sufficient to form respiratory organoids; and (ii) respiratory microorganisms that sustain serial propagation in the respiratory organoids.

The foregoing description of the specific embodiments will so fully reveal the general nature of the disclosure that others can, by applying knowledge within the skill of the relevant art(s) (including the contents of the documents cited and incorporated by reference herein), readily modify and/or adapt for various applications such specific embodiments, without undue experimentation, without departing from the general concept of the present disclosure. Such adaptations and modifications are therefore intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one skilled in the relevant art(s).

While various embodiments of the present disclosure have been described above, it should be understood that they have been presented by way of examples, and not limitation. It would be apparent to one skilled in the relevant art(s) that various changes in form and detail could be made therein without departing from the spirit and scope of the disclosure. Thus, the present disclosure should not be limited by any of the above-described exemplary embodiments but should be defined only in accordance with the following claims and their equivalents.

All references cited herein are incorporated herein by reference in their entirety and for all purposes to the same extent as if each individual publication or patent or patent application was specifically and individually indicated to be incorporated by reference in its entirety for all purposes.

### REFERENCES

1 To, K. K. W., Yip, C. C. Y. & Yuen, K.-Y. Rhinovirus - From bench to bedside. Journal of the Formosan Medical Association 116, 496-504 (2017).
2 Dominguez, S. R. et al. Multiplex MassTag-PCR for respiratory pathogens in pediatric nasopharyngeal washes negative by conventional diagnostic testing shows a high prevalence of viruses belonging to a newly recognized rhinovirus clade. Journal of Clinical Virology 43, 219-222 (2008).
3 Cox, D. W. et al. Human rhinovirus species C infection in young children with acute wheeze is associated with increased acute respiratory hospital admissions. Am J Respir Crit Care Med 188, 1358-1364 (2013).
4 Bochkov, Y. A. et al. Molecular modeling, organ culture and reverse genetics for a newly identified human rhinovirus C. NatMed 17, 627-632 (2011).
5 Ashraf, S., Brockman-Schneider, R., Bochkov, Y. A., Pasic, T. R. & Gern, J. E. Biological characteristics and propagation of human rhinovirus-C in differentiated sinus epithelial cells. Virology 436, 143-149 (2013).
6 Bochkov, Y. A. et al. Cadherin-related family member 3, a childhood asthma susceptibility gene product, mediates rhinovirus C binding and replication. Proc Natl Acad Sci USA 112, 5485-5490 (2015).
7 Griggs, T. F. et al. Rhinovirus C targets ciliated airway epithelial cells. Respir Res 18, 84 (2017).
8 Hao, W. et al. Infection and propagation of human rhinovirus C in human airway epithelial cells. J Virol 86, 13524-13532 (2012).
9 Zhu, N. et al. A Novel Coronavirus from Patients with Pneumonia in China, 2019. N Engl J Med 382, 727-733 (2020).
10 Hou, Y. J. et al. SARS-CoV-2 Reverse Genetics Reveals a Variable Infection Gradient in the Respiratory Tract. Cell 182, 429-446 e414 (2020).
11 Dijkman, R., Koekkoek, S. M., Molenkamp, R., Schildgen, O. & van der Hoek, L. Human bocavirus can be cultured in differentiated human airway epithelial cells. J Virol 83, 7739-7748 (2009).
12 Pyrc, K. et al. Culturing the unculturable: human coronavirus HKU1 infects, replicates, and produces progeny virions in human ciliated airway epithelial cell cultures. J Virol 84, 11255-11263 (2010).
13 Zhou, J. et al. Differentiated human airway organoids to assess infectivity of emerging influenza virus. Proc Natl Acad Sci USA 115, 6822-6827 (2018).
14 Mallapaty, S. The mini lungs and other organoids helping to beat COVID. Nature 593, 492-494 (2021).
15 Li, C. et al. Establishing Human Lung Organoids and Proximal Differentiation to Generate Mature Airway Organoids. J Vis Exp, e63684 (2022).
16 Chiu, M. C. et al. A bipotential organoid model of respiratory epithelium recapitulates high infectivity of SARS-CoV-2 Omicron variant. Cell Discovery (2022).
17 Chiu, M. C. et al. Human Nasal Organoids Model SARS-CoV-2 Upper Respiratory Infection and Recapitulate the Differential Infectivity of Emerging Variants. mBio 13, e0194422 (2022).
18 Chiu, M. C. et al. Establishing Bipotential Human Lung Organoid Culture System and Differentiation to Generate Mature Alveolar and Airway Organoids. Bio Protoc 13, e4657 (2023).
19 Chiu, M. C. et al. Apical-Out Human Airway Organoids Modeling SARS-CoV-2 Infection. Viruses 15 (2023).
20 Chiu, M. C. et al. A bipotential organoid model of respiratory epithelium recapitulates high infectivity of SARS-CoV-2 Omicron variant. Cell Discov 8, 57 (2022).
21 Wang, D. et al. SPINK6 inhibits human airway serine proteases and restricts influenza virus activation. EMBO Mol Med 14, e14485 (2022).
22 Zhou, J. et al. Human intestinal tract serves as an alternative infection route for Middle East respiratory syndrome coronavirus. Sci Adv 3, eaao4966 (2017).
23 Clevers, H. COVID-19: organoids go viral. Nature Reviews Molecular Cell Biology 21, 355-356 (2020).
24 Palmenberg, A. C. & Gern, J. E. Classification and evolution of human rhinoviruses. Methods Mol Biol 1221, 1-10 (2015).
25 Wack, A., Terczynska-Dyla, E. & Hartmann, R. Guarding the frontiers: the biology of type III interferons. Nat Immunol 16, 802-809 (2015).
26 Zhou, J. et al. Infection of bat and human intestinal organoids by SARS-CoV-2. Nat Med 26, 1077-1083 (2020).
27 Li, C. et al. Human airway and nasal organoids reveal escalating replicative fitness of SARS-CoV-2 emerging variants. Proc Natl Acad Sci USA 120, e2300376120 (2023).
28 Liu, X. et al. Analogous comparison unravels heightened antiviral defense and boosted viral infection upon immunosuppression in bat organoids. Signal Transduct Target Ther 7, 392 (2022).
29 Zhao, X. et al. Human Intestinal Organoids Recapitulate Enteric Infections of Enterovirus and Coronavirus. Stem Cell Reports 16, 493-504 (2021).
30 Sanchez, G. A. M. et al. JAK1/2 inhibition with baricitinib in the treatment of auto inflammatory interferonopathies. J Clin Invest 128, 3041-3052 (2018).
31 Travaglini, K. J. et al. A molecular cell atlas of the human lung from single-cell RNA sequencing. Nature 587, 619-625 (2020).
32 Baggen, J., Thibaut, H. J., Strating, J. & van Kuppeveld, F. J. M. The life cycle of non-polio enteroviruses and how to target it. Nat Rev Microbiol 16, 368-381 (2018).
33 Bochkov, Y. A. et al. Mutations in VP1 and 3A proteins improve binding and replication of rhinovirus C15 in HeLa-E8 cells. Virology 499, 350-360 (2016).
34 Li Cun et al. Human Respiratory organoids sustained reproducible propagation of human rhinovirus C and elucidation of virus-host interaction. Nature Communication 15:10772 (2024).

## Claims

1. A composition for culturing a respiratory microorganism, the composition comprising: (i) respiratory organoids selected from the group consisting of airway organoids and nasal organoids; and (ii) a respiratory microorganism, wherein the respiratory microorganism sustains serial propagation in the respiratory organoids.

2. The composition of claim 1, wherein the respiratory organoids are airway organoids derived from lung tissue or are nasal organoids derived from nasal epithelial cells.

3. The composition of claim 1 or claim 2, further comprising an immunosuppressive compound, optionally wherein the immunosuppressive compound interferes with an anti-microorganism response in the respiratory organoids.

4. The composition of claim 3, wherein the immunosuppressive compound is a JAK1/JAK2 inhibitor or a TBK1/IKKε inhibitor, optionally wherein the immunosuppressive compound is Momelotinib (CYT387), Amlexanox, BX795, MRT67307, Ruxolitinib or a combination thereof

5. The composition of any preceding claim, wherein the respiratory microorganism is a respiratory virus or a respiratory bacteria, optionally wherein the respiratory virus is HRV-C, HRV-C3, HRV-C8, HRV-C11, HRV-C15, HRV-C45, influenza virus, adenovirus, human bocavirus, human coronavirus including SARS-CoV1 and SARS-CoV2, human metapneumovirus, human parainfluenza virus, human respiratory syncytial virus, or human rhinovirus; or, optionally, wherein the respiratory bacteria is *Mycobacterium tuberculosis, Streptococcus pneumoniae, Mycoplasma pneumoniae, Hemophilus influenzae, Chlamydophila pneumoniae, Chlamydia psittaci, Coxiella burnetiid, Legionella pneumophila, Staphylococcus aureus,* or *Klebsiella pneumoniae.*

6. The composition of any preceding claim, wherein
the respiratory organoids are human respiratory organoids comprising human respiratory epithelial cells;
the respiratory organoids comprise human lung epithelial cells or human nasal epithelial cells; and/or
the respiratory organoids are derived from human epithelial stem cells.

7. A method for culturing respiratory microorganisms comprising: (i) preparing respiratory organoids, wherein the respiratory organoids are selected from the group consisting of airway organoids and nasal organoids; and (ii) infecting the respiratory organoids with a microorganism, wherein the microorganism sustains propagation in the respiratory organoid.

8. The method of claim 7, further comprising a step of isolating and cultivating the respiratory microorganisms.

9. The method of claim 7 or claim 8, wherein the respiratory organoids are airway organoids derived from lung tissue or are nasal organoids derived from nasal epithelial cells.

10. The method of any of claim 7 to 9, wherein the respiratory organoids are prepared by (a) providing a fragment of an airway tissue or nasal tissue; and (b) culturing the fragment of airway or nasal tissue in a culture medium for a period of time sufficient to form the respiratory organoids, optionally wherein the culture medium further comprises an immunosuppressive compound.

11. The method of claim 10, wherein the immunosuppressive compound interferes with an anti-microorganism response in the respiratory organoids, optionally wherein the immunosuppressive compound is a JAK1/JAK2 inhibitor or a TBK1/IKKε inhibitor, further optionally wherein the immunosuppressive compound is Momelotinib (CYT387).

12. The method of any of claims 7 to 11, wherein the respiratory microorganism sustains at least 3-6 passages with genomic stability.

13. The method of any of claims 7 to 12, wherein the respiratory microorganism is a respiratory virus or a respiratory bacteria, optionally wherein the respiratory virus is HRV-C, HRV-C3, HRV-C8, HRV-C11, HRV-C15, HRV-C45, influenza virus; adenovirus; human bocavirus; human coronavirus including SARS-CoV1 and SARS-CoV2; human metapneumovirus; human parainfluenza virus; human respiratory syncytial virus; and/or human rhinovirusor a combination thereof, or, optionally wherein the respiratory bacteria is *Mycobacterium tuberculosis, Streptococcus pneumoniae, Mycoplasma pneumoniae, Haemophilus influenzae, Chlamydophila pneumoniae; Chlamydia psittaci; Coxiella burnetiid; Legionella pneumophila, Staphylococcus aureus;* and/or *Klebsiella pneumoniae.*

14. The method of any of claims 7 to 13, wherein the respiratory organoids are human respiratory organoids comprising human respiratory epithelial cells.

15. The composition or method of any preceding claim, wherein the respiratory organoids are viable in culture for a period of at least 30 days.

16. A system for culturing respiratory microorganisms comprising: (i) a fragment of lung tissue in a culture medium comprising a JAK1/JAK2 inhibitor or a TBK1/IKKε inhibitor cultured for a period of time sufficient to form respiratory organoids; and (ii) respiratory microorganisms that sustain serial propagation in the respiratory organoids.
